**Europäisches Patentamt**

**European Patent Office**

**Office européen des brevets**

(19)

(11) Publication number: **0 341 675 B1**

# EUROPEAN PATENT SPECIFICATION

(45) Date of publication of patent specification: **03.08.94**    (51) Int. Cl.⁵: **G01N 27/22**, //G01N33/28

(21) Application number: **89108345.3**

(22) Date of filing: **09.05.89**

(54) **Alcohol sensor.**

(30) Priority: **12.05.88 JP 63780/88 U**

(43) Date of publication of application:
**15.11.89 Bulletin 89/46**

(45) Publication of the grant of the patent:
**03.08.94 Bulletin 94/31**

(84) Designated Contracting States:
**DE FR GB**

(56) References cited:
**EP-A- 0 141 636**
**DE-A- 925 621**
**DE-U- 8 609 566**
**FR-A- 2 381 307**
**US-A- 4 757 252**

**PATENT ABSTRACTS OF JAPAN, vol. 5, no. 175 (P-88)[847], 11th November 1981;& JP-A-56 104 240**

(73) Proprietor: **MITSUBISHI DENKI KABUSHIKI KAISHA**
**2-3, Marunouchi 2-chome**
**Chiyoda-ku**
**Tokyo 100(JP)**

(72) Inventor: **Maekawa, Hiroko Mitsubishi Denki K. K.**
**Himeji Seisakusho**
**840, Chiyodacho**
**Himeji-shi Hyogo, 670(JP)**
Inventor: **Suzuki, Hiroyoshi Mitsubishi Denki K. K.**
**Himeji Seisakusho**
**840, Chiyodacho**
**Himeji-shi Hyogo, 670(JP)**
Inventor: **Ogawa, Kenji Mitsubishi Denki K. K.**
**Himeji Seisakusho**
**840, Chiyodacho**
**Himeji-shi Hyogo, 670(JP)**

(74) Representative: **Strehl Schübel-Hopf Groening & Partner**
**Maximilianstrasse 54**
**D-80538 München (DE)**

## Description

The present invention relates to an alcohol sensor of the electrostatic capacity type for detecting a mixing ratio of alcohol in fuel.

Alcohol sensors conventionally adopted for measuring a mixing ratio of alcohol in fuel are those which make use of the fact that an electrostatic capacity formed between a pair or a plurality of pairs of electrode plates disposed in a fuel passage has a proportional relationship to such alcohol mixing ratio. An exemplary one of such alcohol sensors is disclosed, for example, in Japanese Patent Laid-open No. 62-18004.

In such an alcohol sensor of the electrostatic capacity type as described above, a change in electrostatic capacity is converted into a change in oscillation frequency of a CR oscillator, and an alcohol mixing ratio is detected in accordance with a relationship thereof to the oscillation frequency.

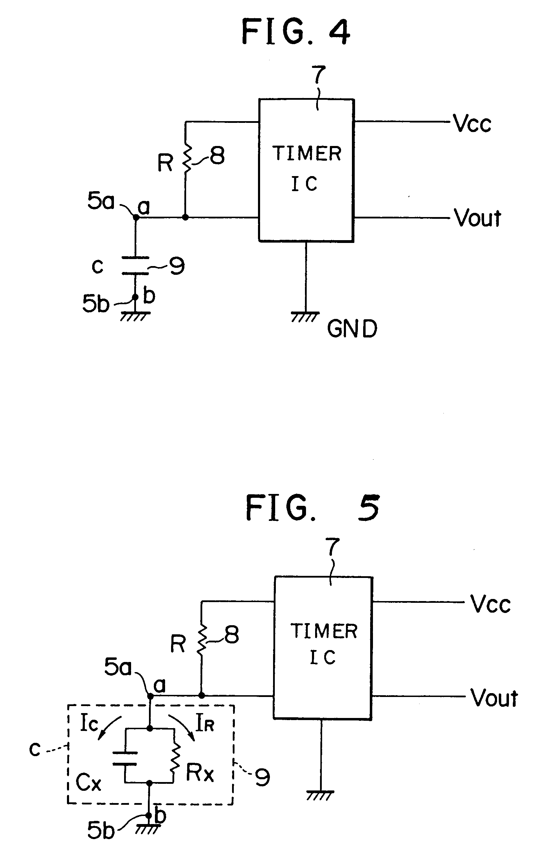

Such a conventional CR oscillating circuit for measurement of an electrostatic capacity is illustratively shown in Fig.4. Referring to Fig.4, the electrostatic capacity measuring CR oscillating circuit shown includes a resistor 8 and a capacitor 9 formed from a pair or a plurality of pairs of electrode plates disposed in a fuel passage. The opposite ends of the capacitor 9 are connected at a pair of points a and b to a timer IC (integrated circuit) 7 by way of a pair of lead wires 5a and 5b, respectively. A resistor 8 is connected to the capacitor 9 at the point a and also connected at the opposite ends thereof to the timer IC 7. The timer IC 7 includes an oscillating element not shown therein and produces oscillations which have a frequency determined by a time constant RC defined by a capacity of the capacitor 9 and a resistance R of the resistor 8.

Actually, the capacitor 9 formed between the electrodes is represented as a parallel circuit of a capacitor having an electrostatic capacity Cx and a resistor having a loss Rx as shown in Fig.5. However, where the loss Rx is so high that it does not have a significant influence on the time constant RC mentioned above, the capacitor C may be considered in such a simplified form as shown in Fig.4. It is to be noted that the electrostatic capacity Cx and the loss Rx shown in Fig.5 are represented by the following equations:

$$Cx = \epsilon * S/d \quad (1)$$

$$Rx = \rho * S/d \quad (2)$$

Here, $\epsilon$ is a dielectric constant of fuel, S an area of opposing faces of the electrode plates, d a distance between the electrodes, and $\rho$ a volume resistivity.

Since the conventional electrostatic capacity measuring CR oscillating circuit has such a construction as described above, if the volume resistivity of fuel decreases due to an increase in concentration of alcohol in gasoline or due to an increase in percentage of water content in gasoline, then the loss Rx of the capacitor 9 decreases. Accordingly, this causes a problem that oscillation of the electrostatic capacity measuring CR oscillating circuit may possibly be stopped.

The reason for this seems to be as follows. In particular, electric current I flowing through the capacitor 9 is divided as Ic and Ir into the electrostatic capacity Cx and the loss Rx, but if the volume resistivity of fuel decreases so that the loss Rx decreases, then the electric current Ir becomes extremely great comparing with the electric current Ic so that the electrostatic capacity Cx finally loses its function. It can be considered that oscillation of the CR oscillator is stopped by this reason.

In EP-A-0 141 636 is disclosed a capacitor formed by two concentric cylinders which are coated with a plastic laminate for electrical insulation from each other. This may enable a measurement of the capacity without relying upon a resistivity of the fluid but requires a complicated process of forming and coating of the capacitor electrodes. Especially, the coating is applied using a high vacuum chamber which requires a high number of working steps and involves high manufacturing costs. The electrodes are fixed in the fluid passage by way of seperate centralisers which are complicated in structure and thus further increase the complexity of the known sensor device.

The reference of patent abstracts of Japan, vol. 5, Nr. 175, P-88, JP-A-56 104 240 refers to an alcohol sensor and has electrodes of plate-form. This embodiment comprises two electrode plates provided in fuel passage forming a capacity but pays no attention to the structure of the electrode plates and the attachment of the electrode plates in the fuel passage.

It is an object of the present invention to provide an electrostatic capacity detecting section of simplified structure which enables a simplification and reduction in working steps for production of an alcohol sensor and is advantageous in cost.

It is a further object to provide an alcohol sensor which can always measure a mixing ratio of alcohol in fuel even if the volume resistivity of the fuel decreases due to an increase in concentration of alcohol in gasoline or due to an increase in percentage of water content in gasoline.

These objects are met by the characterizing features of claim 1.

The alcohol sensor according to the present invention detects a mixing ratio of alcohol in fuel by detecting an electrostatic capacity of a capacitor which is formed by alcohol-mixed fuel filled between the electrode layers covered with electric insulating layers.

With the alcohol sensor, an electrostatic capacity corresponding to a mixing ratio of alcohol can be always detected without relying upon a volume resistivity of fuel and can be coverted into an oscillation frequency with a simple circuit.

The above and other objects, features and advantages of the present invention will become apparent from the following description and the appended claims, taken in conjunction with the accompanying drawings.

Fig.1 is a sectional view of an alcohol sensor showing a preferred embodiment of the present invention;

Fig.2 is a circuit diagram of an electrostatic capacity measuring CR oscillating circuit which employs the alcohol sensor of Fig.1 therein;

Fig.3 is a schematic view illustrating a principle of detection of a mixing ratio of alcohol in fuel by means of a capacitor of the electrostatic capacity measuring CR oscillating circuit of Fig.2;

Fig.4 is a view similar to Fig.2 but showing a conventional electrostatic capacity measuring CR oscillating circuit; and

Fig.5 is a similar view but illustrating a reason for the fact that oscillation is stopped when the loss of a capacitor in the electrostatic capacity measuring CR oscillating circuit of Fig.5 decreases.

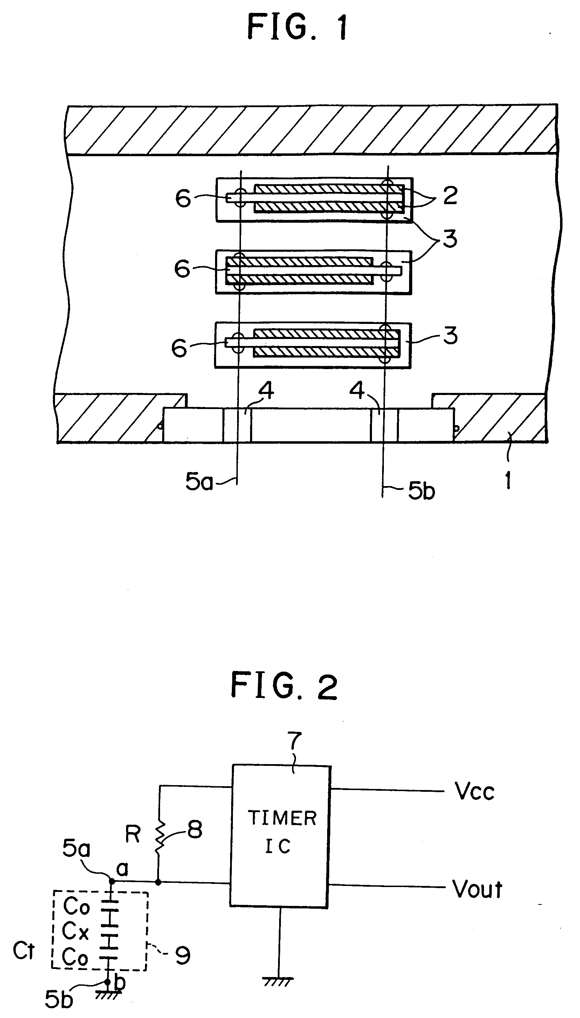

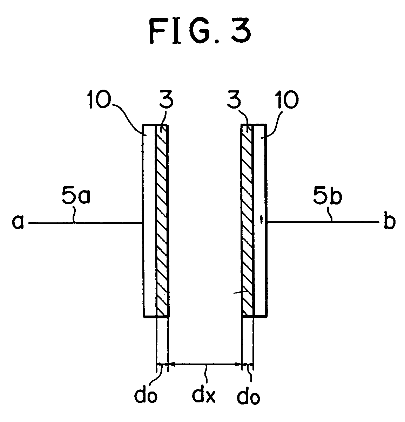

Referring first to Fig.1, there is shown an alcohol sensor according to a preferred embodiment of the present invention. It is to be noted that, in Fig.1 and also in Figs.2 and 3, like parts or elements are denoted by the same reference characters as Figs. 4 and 5 described hereinabove.

The alcohol sensor shown includes a plurality of, three in the present embodiment, base plates 6 each having a pair of electrode layers 2 formed on the opposite surfaces thereof by printing in such a manner as to partially expose the opposite surfaces of the base plates 6. A pair of lead wires 5a and 5b are mechanically coupled to all of the base plates 6 and electrically connected to the electrode layers 2 on different alternate ones of the base plates 6 as seen in Fig.1. Surfaces of each of the base plates 6 and the electrode layers 2 on each base plate 6 are covered with an electric insulating layer 3 so that neither of the base plates 6 nor the electrode layers 2 may be exposed. In this arrangement, printing of an electric insulating material may be employed in addition to physico-chemical means as described below in order to form such electric insulating layers 2. Such printing technique will enable simplification and reduction in working steps for production of an alcohol sensor and is advantageous in cost.

The lead wires 5a and 5b are mechanically coupled to the electrode layers 2 while assuring electric insulation between the electrode layers 2 and the lead wires 5a and 5b. The lead wires 5a and 5b are connected at a pair of points a and b to an electrostatic capacity measuring CR oscillating circuit shown in Fig.2. The lead wires 5a and 5b thus support the electrode layers 2 in position thereon in the fuel passage 1 due to their rigidity.

All surfaces of the elements of the alcohol sensor described above are covered with an electric insulating layer 3 in such a manner that only the electric insulating layer 3 is exposed to fuel in the fuel passage 1. A capacitor 9 shown in Fig.2 for detecting alcohol is thus formed with portions of the electric insulating layer 3 located between the individual electrode layers 2.

Referring now to Fig.2, there is shown an electrostatic capacity measuring CR oscillating circuit which employs the alcohol sensor of Fig.1. The electrostatic capacity measuring CR oscillating circuit shown has a substantially similar construction to that of the conventional electrostatic capacity measuring CR oscillating circuit shown in Fig.5 but is only different in detailed construction of the capacitor 9 serving as an alcohol sensor. In particular, the opposite ends of the capacitor 9 are connected at a pair of points a and b to a timer IC (integrated circuit) 7 by way of a pair of lead wires 5a and 5b, respectively. A resistor 8 is connected to the capacitor 9 at the point a and also connected at the opposite ends thereof to the timer IC 7 which includes an oscillating element therein (not shown).

Referring now to Fig.3, there is illustrated a principle of the capacitor 9 shown in Fig.2. In particular, a capacitor 9 formed from a pair of electrode plates 10 opposing surfaces of which are covered with electric insulating layers 3 has an electrostatic capacity C which is equivalent to that of a series circuit of a capacitor having an electrostatic capacity $C_x$ formed by fuel filled between the electrode plates 10 and another capacitor having an electrostatic capacity $C_o$ formed by the electric insulating layers 3. In this instance, the composite electrostatic capacity $C_t$ of the electrostatic capacity measuring CR oscillating circuit is represented by the following equation.

$$C_t = 1/(2/C_o + 1/C_x) \qquad (3)$$

Since the oscillation frequency of the CR oscillating circuit of Fig.2 is determined in accordance with a time constant defined by a resistance R of the resistor 8 and the composite electrostatic capacity $C_t$ of the capacitor 9, if the electrostatic capacity $C_o$ is made as high as possible, $C_t = C_x$ is almost reached as apparent from the equation (3). In this instance, no influence of the electric insulating layers 3 is provided on the composite electrostatic capacity Ct of the capacitor 9.

Accordingly, a relationship Co ≫ Cx should be established in the equation (3) above. To this end, the inter-electrode distance do of the electrostatic capacity Co, that is, the thickness do of each electric insulating layer 3, should be set sufficiently smaller than the inter-electrode distance dx of the electrostatic capacity Cx, or else a material of a high dielectric constant should be employed for the electric insulating layers 3 so as to assure a high dielectric constant $\epsilon$.

In this instance, even if fuel has a low volume resistivity, oscillation of the electrostatic capacity measuring CR oscillating circuit is not stopped because charging and discharging occur at the electrostatic capacity Ct due to the fact that charging and discharging occur at the electrostatic capacity Co of the capacitor 9. In other words, where the electrode plates 10 are covered with the electric insulating layers 3, the alcohol sensor can always convert a change in characteristics of fuel into a change oscillation frequency.

Referring back to Fig.1, the alcohol sensor shown can be produced by such a method that an electric insulating material is coated on a surface of an electrode layer 2 by a physico-chemical means such as painting, adhesion, welding, evaporation and dipping to form an electric insulating layer 3 and then such electrode plates are assembled into such an alcohol sensor as shown in Fig.1. The alcohol sensor may be produced otherwise by another method that an assembly of such electrode layers 2 as shown in Fig.1 is first produced and then an electric insulating material is coated on all of electrostatic capacity detecting sections of the assembly by such physico-chemical means as listed above to form such an electric insulating layer or layers 3 as seen in Fig.1

While in the alcohol sensor of the embodiment shown in Fig.1 the surfaces of the electrostatic capacity detecting sections to be exposed in the fuel passage 1 are entirely covered with the electric insulating layer or layers 3, they may otherwise be covered partially or in a different manner with the electric insulating layer or layers 3.

It is to be noted that, while in the embodiments of Figs.1 the two capacitors are connected in parallel, the number of such capacitors may be selected suitably by a user depending upon an area of and a distance between the electrodes, a dielectric constant $\epsilon$ of the electric insulating layers 3 and the oscillation frequency of the electrostatic capacity measuring CR oscillating circuit.

Further, only if a surface of an electrostatic capacity detecting section is covered with an electric insulating layer, similar effects can be anticipated without depending upon the configurations of the embodiments and the coating means. Where a sensor is accommodated in a case, if the case itself is covered with an electric insulating layer, then the influence of a resistance provided between the case and electrodes can be ignored by the same reason as in the embodiments described above, and accordingly, improvement in accuracy can be obtained.

## Claims

1. An alcohol sensor for measuring a ratio of alcohol contained in a fuel fed through a fuel passage (1), said alcohol sensor comprising;

   an electrostatic capacity detecting section (CR) disposed in said fuel passage (1) to measure an electric capacity of the fuel fed therethrough;

   a plurality of electrode plates (6, 10); and

   a pair of lead wires (5a, 5b) for connecting said electrode plates (6, 10) in said fuel passage (1) to thereby form said electrostatic capacity detecting section;

   characterized in that each of said electrode plates (6, 10) is formed with a pair of electrode layers (2) printed on the opposite surfaces thereof; said pair of lead wires (5a, 5b) being mechanically coupled to all of said electrode plates (6, 10) and electrically connected to said electrode layers (2) on different alternate ones of said electrode plates (6, 10), wherein all surfaces of each of said electrode layers (2) are covered with an electrically insulating layer (3) printed thereon, the thickness of each of said electrically insulating layers (3) is significantly smaller than a distance (dx) between said electrode plates (6, 10) and said electrode plates (6, 10) are supported in said fuel passage (1) by way of said lead wires (5a, 5b) due to rigidity thereof.

2. The alcohol sensor of claim 1, wherein said electric insulating layers (3) are made of a material having a high dielectric constant.

## Patentansprüche

1. Alkoholsensor zur Messung des Alkoholanteils eines Brennstoffs, der durch eine Brennstoffleitung (1) geleitet wird, umfassend:

   einen elektrostatisch-kapazitiven Erfassungsabschnitt (CR), der in der Brennstoffleitung (1) zur Messung der elektrostatischen Kapazität des durchgeleiteten Brennstoffs angebracht ist;

   mehrere Elektrodenplatten (6, 10); und

   ein Anschlußleitungspaar (5a, 5b) zum Anschluß der Elektrodenplatten (6, 10) in der Brennstoffleitung (1), um den elektrostatisch-kapazitiven Erfassungsabschnitt zu bilden;

dadurch gekennzeichnet, daß jede der Elektrodenplatten (6, 10) mit einem Paar von auf den gegenüberliegenden Oberflächen aufgedruckten Elektrodenschichten (2) gebildet ist; wobei das Anschlußleitungspaar (5a, 5b) mit allen Elektrodenplatten (6, 10) mechanisch gekoppelt ist und alternierend mit den Elektro-denschichten (2) auf verschiedenen Elektro-denplatten (6, 10) verbunden ist, wobei alle Flächen von jeder Elektrodenschicht (2) mit einer aufgedruckten elektrisch isolierenden Schicht (3) bedeckt sind, und die Dicke jeder isolierenden Schicht (3) erheblich geringer ist ist als die Entfernung (dx) zwischen den Elektrodenplatten (6, 10), und die Elektrodenplatten (6, 10) in der Brennstoffleitung (1) mittels der Anschlußleitungen (5a, 5b) durch deren Steifigkeit gehalten sind.

2. Alkoholsensor nach Anspruch 1, wobei die elektrisch isolierenden Schichten (3) aus einem Material mit einer hohen Dielektrizitätskonstanten gebildet ist.

## Revendications

1. Détecteur d'alcool pour mesurer un rapport d'alcool contenu dans un carburant délivré par l'intermédiaire d'un conduit de carburant (1), ledit détecteur d'alcool comprenant :

un module de détection de capacité électrostatique (CR) disposé dans ledit conduit de carburant (1) pour mesurer la capacité électrostatique du carburant qui le traverse ;

une pluralité de plaques électrodes (6, 10) ; et

une paire de fils conducteurs (5a, 5b) pour connecter lesdites plaques électrodes (6, 10) dans ledit conduit de carburant (1) pour former ainsi ledit module de détection de capacité électrostatique ;

caractérisé en ce que chacune desdites plaques électrodes (6, 10) est formée d'une paire de couches d'électrode (2) imprimées sur ses faces opposées, ladite paire de fils conducteurs (5a, 5b) étant fixée mécaniquement à toutes lesdites plaques électrodes (6, 10) et connectées électriquement auxdites couches d'électrode (2) d'une sur deux desdites plaques électrodes (6, 10) ; dans lequel toutes les surfaces de chacune desdites couches d'électrode (2) sont recouvertes d'une couche isolante de l'électricité (3) imprimée sur celles-ci, l'épaisseur de chacune desdites couches isolantes de l'électricité (3) étant plus petite de manière significative que la distance (dx) entre lesdites plaques électrodes (6, 10), et lesdites plaques électrodes (6, 10) étant supportées dans ledit conduit de carburant (1) au moyen desdits fils conducteurs (5a, 5b) grâce à leur rigidité.

2. Détecteur d'alcool selon la revendication 1, dans lequel lesdites couches isolantes de l'électricité (3) sont faites d'une matière ayant une constante diélectrique élevée.

# FIG. 1

# FIG. 2

# FIG. 3

# FIG. 4

# FIG. 5